(19) **Europäisches Patentamt**
**European Patent Office**
**Office européen des brevets**

(11) **EP 4 146 159 B1**

(12) **FASCICULE DE BREVET EUROPEEN**

(45) Date de publication et mention de la délivrance du brevet:
**07.08.2024   Bulletin 2024/32**

(21) Numéro de dépôt: **21723871.6**

(22) Date de dépôt: **04.05.2021**

(51) Classification Internationale des Brevets (IPC):
*A61K 8/67* *(2006.01)*    *A61Q 19/08* *(2006.01)*

(52) Classification Coopérative des Brevets (CPC):
**A61K 8/675; A61Q 19/08;** A61K 2800/81

(86) Numéro de dépôt international:
**PCT/EP2021/061729**

(87) Numéro de publication internationale:
**WO 2021/224259 (11.11.2021 Gazette 2021/45)**

(54) **MÉTHODE DE TRAITEMENT COSMÉTIQUE PAR ILLUMINATION ET APPLICATION COMBINÉE D'UNE COMPOSITION COMPRENANT DE LA NIACINAMIDE, ET DISPOSITIF ASSOCIÉ**

VERFAHREN ZUR KOSMETISCHEN BEHANDLUNG DURCH BELEUCHTUNG UND KOMBINIERTE ANWENDUNG EINER NIACINAMID ENTHALTENDEN ZUSAMMENSETZUNG UND VORRICHTUNG DAFÜR

METHOD OF COSMETIC TREATMENT BY ILLUMINATION AND COMBINED APPLICATION OF A COMPOSITION COMPRISING NIACINAMIDE, AND DEVICE THEREFOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **06.05.2020  FR 2004500**

(43) Date de publication de la demande:
**15.03.2023   Bulletin 2023/11**

(73) Titulaire: **Lightinderm**
**75017 Paris (FR)**

(72) Inventeurs:
- **DECLERCQ, Lieve**
  **9070 Destelbergen (BE)**
- **GUERMONPREZ, Cyprien**
  **75011 Paris (FR)**

(74) Mandataire: **Delaveau, Sophie**
**ETNA**
**3, rue Geoffroy Marie**
**75009 Paris (FR)**

(56) Documents cités:
EP-A1- 3 586 926      WO-A1-2016/146778
US-A1- 2014 236 265    US-A1- 2018 200 174

**Description**

**DOMAINE TECHNIQUE**

L'invention s'inscrit dans le domaine des traitements cosmétiques non thérapeutiques par illumination de la peau. L'invention s'inscrit en outre dans le domaine des traitements cosmétiques par application d'une composition comprenant au moins un actif.

L'invention porte en outre sur un dispositif de mise en oeuvre d'un tel procédé.

**ART ANTERIEUR**

Pour limiter les effets du vieillissement sur la peau du corps ou du visage tels que les rides, la densité du derme, la luminosité et l'homogénéité du teint, il est connu d'appliquer des compositions de type crème comportant un ou plusieurs agents actifs. Ces compositions présentent le plus souvent un effet limité mais surtout, les effets ne se révèlent qu'à l'issu d'une période plus ou moins longues de traitement.

Il est connu de combiner l'application d'une composition avec une illumination pour accélérer l'effet des agents actifs appliqués sur la peau.

A titre d'exemple, la publication US2008031833 concerne un procédé pour améliorer l'état de la peau et pour retarder et/ou prévenir les signes de son vieillissement. Ce procédé consiste en l'application d'une composition dont l'agent actif est choisi parmi une multitude d'actifs de soin, et d'une illumination qui peut être en mode continu ou pulsé, et qui balaye le spectre UV, le spectre visible et le spectre infrarouge. Mais cette publication ne fait pas état de résultats significatifs sur l'atténuation du vieillissement de la peau.

La publication US2007112042 décrit l'association de l'application d'un vasodilatateur topique, et d'une illumination avec un laser pulsé à 585 nm pour accroitre la production de collagène. Les résultats ne sont pas non plus exposés.

La publication US2009209600 divulgue un dispositif d'application d'un produit pour améliorer l'hydratation de la peau à partir d'une composition cosmétique formulée à partir de niacinamide, le produit étant distribué par un dispositif chauffant constitué par une lampe infrarouge.

La publication EP2861203 divulgue un procédé de traitement cosmétique des peaux grasses dans lequel la peau est soumise à une exposition lumineuse quasi chromatique dans une gamme allant de 300 à 700 nm, et le cas échéant à une seconde exposition lumineuse dans le rouge et l'infrarouge. Il est évoqué l'application simultanée d'une composition à base de particules creuses telles que des particules d'aérogel de silice hydrophobe. Mais cette association agit spécifiquement sur la sécrétion de sébum.

La publication US2018200174 divulgue un procédé de traitement cosmétique agissant notamment comme traitement anti-âge dans lequel est appliqué sur la peau un extrait d'algue ou de plante comprenant une enzyme activable par la lumière, un extrait de graine de baobab, un extrait de Tephrosia purpurea et un extrait de pre- ou probiotiques, complétés par une illumination dans une gamme allant de 630 à 700nm et/ou une gamme allant de 420 à 460nm, cette dernière gamme interagissant plus particulièrement avec l'enzyme.

La publication WO2016/146778 divulgue un dispositif électroluminescent de traitement cutané, et un procédé associé, agissant notamment comme antivieillissement, et incluant une illumination selon trois gammes conjointes de longueurs d'onde: 510-536 nm, 650-670 nm et 768-792 nm. A l'illumination peut s'ajouter l'application d'un agent actif.

La publication EP3586926 divulgue un procédé de traitement antivieillissement de la peau, dans lequel on combine l'application de chlorhydrate de glucosamine avec une illumination dans une gamme allant de 600 nm à 750 nm et/ou de 750 nm à 1000 nm.

La publication US2014236265 divulgue un procédé anti-acnéique dans lequel une composition nettoyante est appliquée sur la peau, puis une illumination dans une gamme allant de 390 à 430 nm est opérée, laquelle illumination est suivie de l'application d'une composition antioxydante pour limiter les effets indésirables sur la peau de la photothérapie. Dans un mode de réalisation particulier, une illumination supplémentaire dans une gamme allant de 600 à 700nm est prévue.

**OBJECTIF DE L'INVENTION**

Il existe ainsi un besoin pour une méthode de traitement cosmétique de la peau qui combine les effets de l'illumination et d'un ou plusieurs agents actifs pour réduire significativement les effets du vieillissement cutané.

A cet effet, l'invention vise une méthode de traitement et un dispositif associé qui atténuent de manière démontrée et visible les principaux effets du vieillissement de la peau à savoir notamment les rides, la perte de densité du derme, la diminution de la luminosité du teint et le manque d'homogénéité du teint.

## EXPOSE DE L'INVENTION

À cet effet, l'invention vise un procédé de traitement cosmétique non thérapeutique comprenant au moins les étapes de :

- application sur la peau d'un sujet d'une composition active comprenant au moins de la niacinamide, et
- illumination de la peau par au moins une source lumineuse émettant à une longueur d'onde comprise entre 620 et 690 nanomètres,

L'illumination étant réalisée avant, simultanément ou après l'application de la composition.

Contrairement aux procédés divulgués dans les documents cités dans l'art antérieur, l'association spécifique de la niacinamide et d'une illumination à une longueur d'onde comprise entre 620 et 690 nanomètres, plus préférentiellement de 660 nanomètres, a permis de mettre en évidence l'effet anti-inflammatoire du procédé de l'invention sur kératinocytes et l'accroissement de la production de pro-collagène 1, attestant ainsi d'un effet antivieillissement de la peau.

La méthode de traitement cosmétique peut également comporter les caractéristiques optionnelles suivantes considérées isolément ou selon toutes les combinaisons techniques possibles :

- la composition active comprend en outre de la photolyase,
- l'illumination de la peau est en outre réalisée par au moins une source lumineuse émettant à une longueur d'onde comprise entre 410 et 470 nanomètre, de préférence de 440 nanomètres,

L'association de la photolyase dans la composition, en plus de la niacinamide, et d'une longueur d'onde comprise entre 410 et 470 nanomètres, plus préférentiellement de 440 nanomètres, a permis de constater de façon surprenante, une réduction de la densité des dimères de thymine exposés aux UV, ainsi qu'une diminution visible des rides, une augmentation de la densité du derme, un effet lissant, et une homogénéisation du teint.

- l'illumination de la peau est en outre réalisée par au moins une source lumineuse émettant à une longueur d'onde comprise entre 750 et 810 nanomètres, de préférence de 780 nanomètres,
- l'illumination de la peau est en outre réalisée par au moins une source lumineuse émettant à une longueur d'onde comprise entre 910 et 970 nanomètres, de préférence de 940 nanomètres,
- le pourcentage massique de niacinamide dans la composition est compris entre 0.5 et 10%, de préférence compris entre 0,5 et 7%, plus préférentiellement entre 3 et 7%.
- le pourcentage massique de photolyase dans la composition est compris entre 0,001 % et 0,01%,
- la composition active comprend du hyaluronate de sodium,
- la composition active comprend un extrait d'algue Laminaria,
- la composition active comprend de la vitamine C,
- la composition active comprend de la vitamine E,
- la composition active comprend en outre de l'acétyle hexapeptide - 8,
- la composition active comprend en outre de la caféine,
- l'illumination est réalisée simultanément à l'application de la lumière sur la peau,
- le spectre de l'illumination est discontinu,
- la durée d'illumination est inférieure à 180 secondes, de préférence inférieure ou égal à 30 secondes.

L'invention vise également un dispositif de traitement cosmétique non thérapeutique comprenant au moins des moyens d'application sur la peau d'un sujet d'une composition active comprenant au moins de la niacinamide, et des moyens d'illumination de la peau par au moins une source lumineuse émettant à une longueur d'onde comprise entre 620 et 690 nanomètres,
les moyens d'illumination et d'application de la composition active étant configurés pour que l'illumination puisse être réalisée avant, simultanément ou après l'application de la composition.

Le dispositif de l'invention peut également comporter les caractéristiques optionnelles suivantes considérées isolément ou selon toutes les combinaisons techniques possibles :

- la composition active comprend en outre de la photolyase,
- les moyens d'illumination de la peau comporte en outre au moins une source lumineuse émettant à une longueur d'onde comprise entre 410 et 470 nanomètres, de préférence de 440 nanomètres,
- les moyens d'illumination de la peau comporte en outre au moins une source lumineuse émettant à une longueur d'onde comprise entre 750 et 810 nanomètres, de préférence de 780 nanomètres,

- les moyens d'illumination de la peau comporte en outre au moins une source lumineuse émettant à une longueur d'onde comprise entre 910 et 970 nanomètre, de préférence de 940 nanomètres.

L'invention porte enfin sur un procédé de traitement cosmétique non thérapeutique comprenant la mise en oeuvre du dispositif précédemment évoqué.

## PRESENTATION DES FIGURES

D'autres caractéristiques et avantages de l'invention ressortiront clairement de la description qui en est donnée ci-dessous, à titre indicatif et nullement limitatif, en référence aux figures annexées parmi lesquelles :

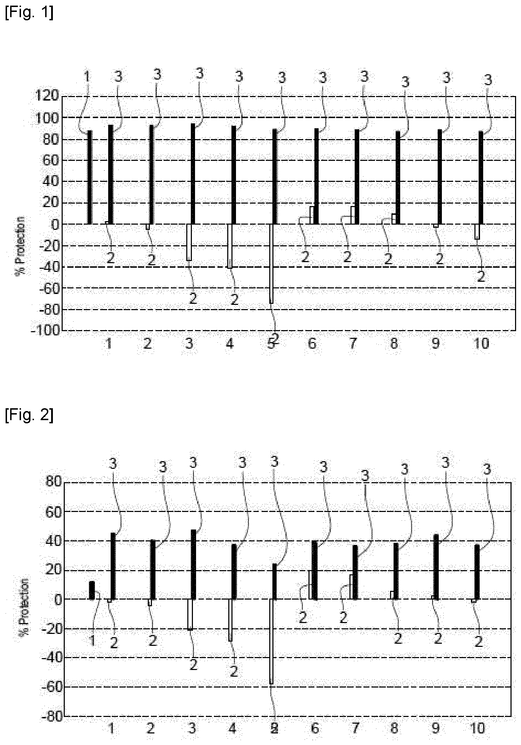

[Fig. 1] La figure 1 est un histogramme illustrant le pourcentage de protection de cellules par le biais de la mesure de la production des interleukines 6, les cellules ayant été préalablement exposées à une combinaison d'UVA et d'UVB et traitées par une illumination seule à une longueur d'onde de 660 nanomètres, par l'application seule de niacinamide, et par le procédé de traitement de l'invention avec une composition comportant de la niacinamide et une illumination discontinue à une longueur d'onde de 660 nanomètres selon 10 programmes d'illumination différents.

[Fig. 2] La figure 2 est un histogramme illustrant le pourcentage de protection de cellules par le biais de la mesure de la production des interleukines 8, les cellules ayant été préalablement exposées à une combinaison d'UVA et d'UVB et traitées par une illumination seule à une longueur d'onde de 660 nanomètres, par l'application seule de niacinamide, et par le procédé de traitement de l'invention avec une composition comportant de la niacinamide et une illumination discontinue à une longueur d'onde de 660 nanomètres selon 10 programmes d'illumination différents, par une illumination seule à une longueur d'onde de 660 nanomètres,

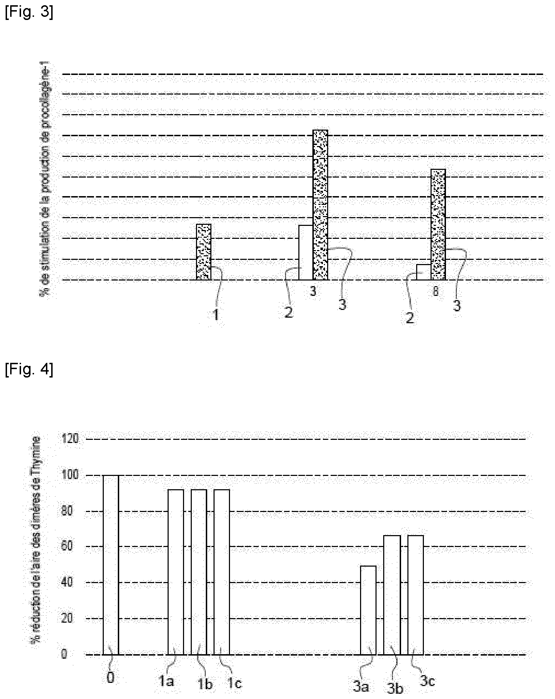

[Fig. 3] La figure 3 est un histogramme illustrant le pourcentage de stimulation de la production de procollagène-1 de cellules préalablement exposées à une combinaison d'UVA et d'UVB et traitées par une illumination seule à une longueur d'onde de 660 nanomètres, par l'application seule de niacinamide, et par le procédé de traitement de l'invention avec une composition comportant de la niacinamide et une illumination discontinue à une longueur d'onde de 660 nanomètres selon 2 programmes d'illumination différents, et par une illumination seule à une longueur d'onde de 660 nanomètres.

[Fig. 4] La figure 4 est un histogramme illustrant le pourcentage de réduction de l'aire des dimères de thymine d'explants de peau préalablement exposées une combinaison d'UVA et d'UVB, dépourvus de traitement, traités par l'application seule d'une composition comprenant de la niacinamide et de la photolyase, et traitées par le procédé de traitement de l'invention avec une composition comportant de la niacinamide et de la photolyase, et une illumination discontinue de longueur d'onde d'au moins 660 nanomètres et 440 nanomètres selon trois programmes différents.

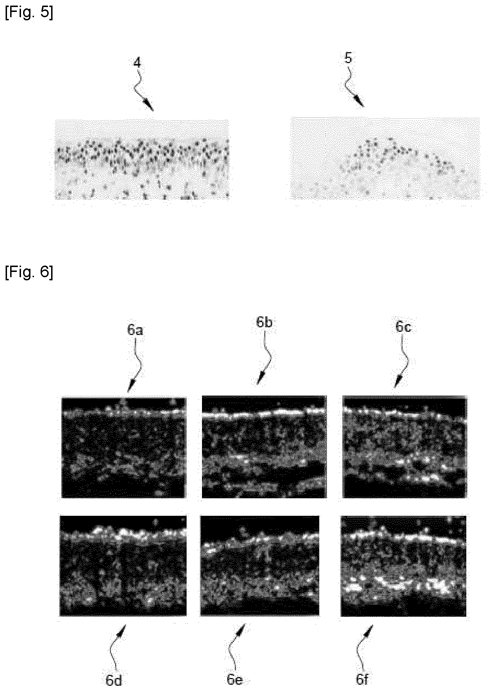

[Fig. 5] La figure 5 illustre deux photographies d'un explant de peau constatant les dégâts par le biais de la présence des dimères de thymine d'une exposition aux UVA et UVB, l'explant de la photographie de gauche n'ayant été soumis à aucun traitement, et l'explant de la photographie de droite ayant été soumis au procédé de traitement de l'invention avec une composition comportant de la niacinamide et de la photolyase, et une illumination discontinue à une longueur d'onde d'au moins 660 nanomètres et 440 nanomètres.

[Fig. 6] La figure 6 illustre six échographies respectivement 6a, 6b, 6c, 6d, 6e, 6f illustrant la densité du derme de deux sujets qui ont bénéficié pendant 28 jours de la méthode de traitement de l'invention avec une composition comportant de la niacinamide et de la photolyase et une illumination discontinue à une longueur d'onde d'au moins 660 nanomètres et 440 nanomètres, en comparaison avec le premier jour de traitement et le 28ème jour d'un traitement par un placebo.

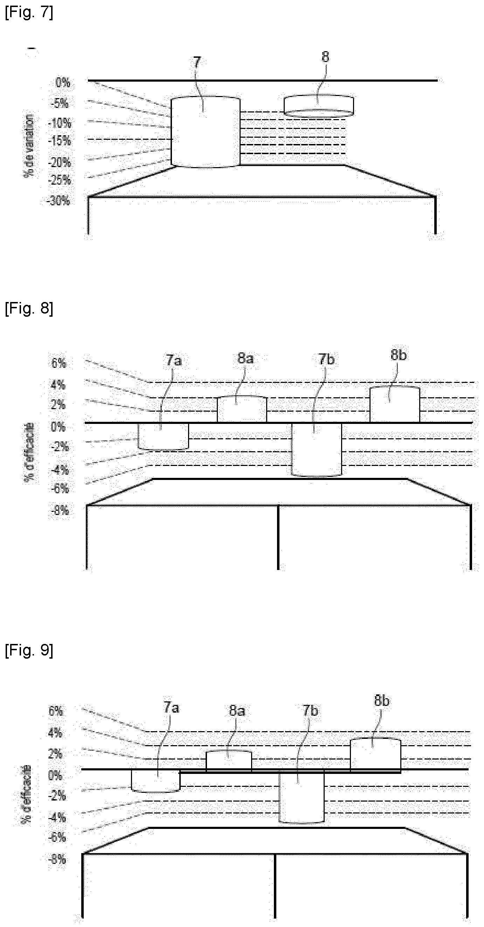

[Fig. 7] La figure 7 est un histogramme comparant schématiquement le pourcentage moyen de réduction de la surface non-échogène de la peau pour tous les sujets traités à 28 jours de traitement, et pour chacun desquels une demi-face du visage est traitée par la méthode de traitement de l'invention avec une composition comportant de la niacinamide et de la photolyase et une illumination discontinue à une longueur d'onde d'au moins 660 nanomètres et 440 nanomètres, en comparaison avec l'autre demi-face du visage traitée par un placebo,

[Fig. 8] [Fig. 9] Les figures 8 et 9 sont des histogrammes comparant schématiquement le pourcentage moyen d'efficacité sur le relief moyen et l'amplitude maximale d'un profil 3D par profilométrie pour tous les sujets traités à 14 jours et 28 jours de traitement et pour chacun desquels une demi-face du visage est traitée par la méthode de traitement de l'invention avec une composition comportant de la niacinamide et de la photolyase et une illumination discontinue à une longueur d'onde d'au moins 660 nanomètres et 440 nanomètres, en comparaison avec l'autre demi-face du visage traitée par un placebo,

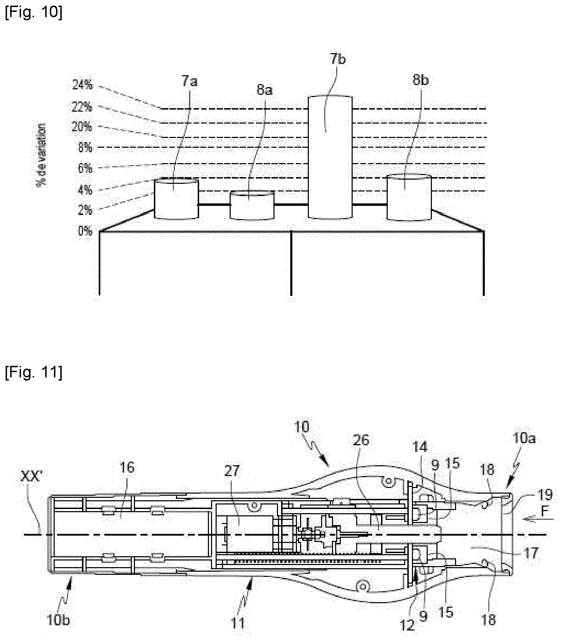

[Fig. 10] La figure 10 est un histogramme comparant schématiquement le pourcentage moyen de variation du lissage de la peau pour tous les sujets traités à 14 jours et 28 jours de traitement et pour chacun desquels une demi-face du visage est traitée par la méthode de traitement de l'invention avec une composition comportant de la niacinamide et de la photolyase et une illumination discontinue à une longueur d'onde d'au moins 660 nanomètres et 440 nanomètres, en comparaison avec l'autre demi-face du visage traitée par un placebo,

[Fig. 11] La figure 11 est une représentation schématique en coupe du dispositif de l'invention dépourvu de sa capsule d'application de la composition,

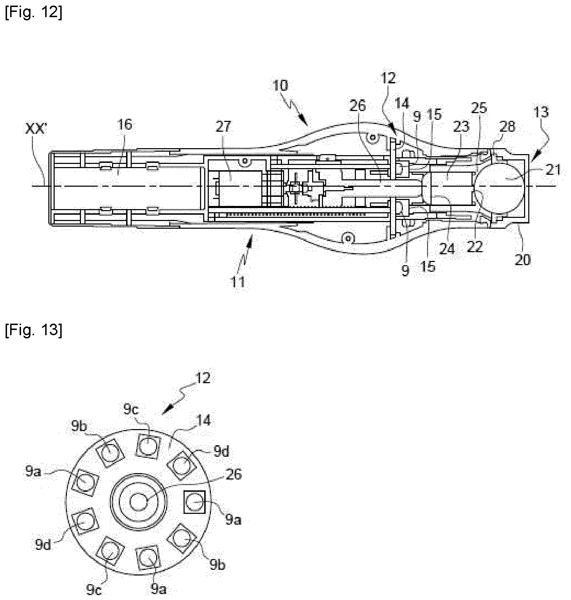

[Fig. 12] La figure 12 est une représentation schématique en coupe du dispositif de l'invention illustré avec la capsule d'application de la composition, et

[Fig. 13] La figure 13 est une représentation schématique de face d'un exemple de réalisation du dispositif électroluminescent du dispositif de l'invention des figures 11 et 12.

## DESCRIPTION DETAILLEE DE L'INVENTION

Le procédé de traitement cosmétique non thérapeutique de l'invention comprend essentiellement une étape d'application sur la peau d'un sujet d'une composition active comprenant au moins de la niacinamide comme agent actif et une illumination, de préférence simultanément, de cette même zone de la peau, par une source lumineuse émettant dans le spectre rouge à une longueur d'onde d'environ 660nm, considérant une largeur de demi-bande de 30nm. Lorsque l'illumination est réalisée avant ou après l'application de la composition, on considère un temps maximum entre l'illumination et l'application de la niacinamide de 30 minutes, de préférence de 15 minutes.

Le procédé de traitement cosmétique de l'invention s'applique exclusivement sur une peau dénuée de plaie ouverte et de toute autre pathologie. Le procédé de l'invention s'applique sur une peau saine, et plus particulièrement sur une peau qui présente des signes de vieillissement essentiellement dus à son exposition au soleil.

Des essais in vitro de mesures de l'effet anti-inflammatoire des cellules de l'épiderme et de l'effet antivieillissement de ces cellules par le procédé de l'invention ont été menés. L'inflammation des cellules et la mesure de l'effet antivieillissement reposent sur l'irradiation d'une culture cellulaire avec une combinaison d'UVA et d'UVB. Ces cellules sont traitées soit par la niacinamide seule, soit par une illumination à 660 nm seule, soit par la combinaison de l'application de niacinamide et de l'illumination à 660 nm selon le procédé de l'invention et selon le protocole suivant :

- au temps T -24h, un traitement est appliqué, étant entendu que ce traitement est l'un des trois traitements comparatifs précédemment mentionné,
- au temps T0, l'irradiation aux UVA et UVB est effectué, suivi du même traitement qu'à T -24h,
- au temps T +24h, le même traitement est appliqué, et
- au temps T +48h, le surnageant est prélevé.

On utilise de la niacinamide pure à une concentration de 0,4 mg/ml ou de 1,2 mg/mL.

Pour les traitements induisant une illumination à 660nm, 10 programmes différents sont évalués. Ces programmes sont définis dans le Tableau 1 ci-dessous.

[Tableaux 1] : Paramètres associés à chaque programme d'illumination

| Programme | 1 | 2 | 3 | 4 | 5 | 6 | 7 | 8 | 9 | 10 |
|---|---|---|---|---|---|---|---|---|---|---|
| Irradiance (mW/cm$^2$) | 4,0 | 26,6 | 10,6 | 10,6 | 2,7 | 10,6 | 10,6 | 5,3 | 10,6 | 10,6 |
| Rapport cyclique (%) | 50 | 50 | 50 | 50 | 50 | 20 | 71,4 | 50 | 50 | 50 |
| Période T (ms) | 14 | 14 | 70 | 350 | 14 | 15 | 14 | 14 | 14 | 14 |
| Fluence (mJ/cm$^2$) | 30 | 199 | 80 | 80 | 80 | 32 | 114 | 80 | 80 | 199 |
| Temps (s) | 15 | 15 | 15 | 15 | 60 | 15 | 15 | 30 | 15 | 37,5 |

En référence aux paramètres indiqués dans le tableau ci-dessus, on apporte les précisions suivantes. L'irradiation réalisée est en mode pulsé et présente un signal rectangulaire. La période T du signal correspond à la somme de la période $T_{on}$ pendant laquelle le signal est à son maximum, et de la période $T_{off}$ pendant laquelle le signal est nul. L'irradiance correspond à la densité de puissance par unité de surface pendant la période $T_{on}$. La fluence correspond à la quantité d'énergie par unité de surface pendant la période $T_{on}$. Le rapport cyclique correspond au rapport entre la période $T_{on}$ et la période T.

A l'issu du protocole précédemment défini, on obtient trois types de surnageants : le premier traité uniquement avec de la niacinamide, le second soumis à une illumination selon l'un des 10 programmes d'illumination à 660 nm, et le troisième soumis à la combinaison de la niacinamide et de l'illumination selon l'un des 10 programmes d'illumination à 660 nm.

On s'intéresse en premier lieu à l'effet anti-inflammatoire du procédé de l'invention sur des cellules de l'épiderme, les kératinocytes. L'irradiation aux UV induit une inflammation des kératinocytes. Les UV sont en effet des rayonnements très énergétiques capables de produire des radicaux libres dans les cellules en rompant des liaisons covalentes de molécules. Les radicaux libres sont réactifs et induisent une cascade inflammatoire caractérisée par la production de protéines pro-inflammatoires (p53, pro-opiomélanocortine (POMC), Interleukines, ...) qu'il est possible de mesurer dans le surnageant des cellules.

Les cytokines communes à large spectre d'inflammations sont les interleukines 6 et 8 (IL-6 et IL-8) qui sont choisies comme paramètres de mesure, étant entendu que les UVA et les UVB induisent des dégâts notamment sur les kératinocytes qui produisent plus d'interleukines 6 et 8 que des cellules non exposées aux UV.

Selon le protocole, à T +48h, le surnageant des cellules est prélevé et les concentrations d'interleukines 6 et 8 sont mesurées et comparées aux concentrations d'interleukines 6 et 8 produites par des cellules soumises exposées aux UV et soumises à aucun des 3 traitement susmentionnés (témoin UV) et à des cellules non traitées et non irradiées (Témoin non irradié). On évalue un taux de protection des cellules traitées par rapport au témoin UV selon la formule suivante :

[Math. 1]

$$\text{Protection (\%)} = \left( \frac{\text{Moyenne Témoin UV} - \text{Concentration IL6 ou IL8}}{\text{Moyenne Témoin UV} - \text{Moyenne Témoin non irradié}} \right) x \ 100$$

Les cellules non irradiées présentent une protection de 100% et les cellules irradiées présentent une protection de 0%. Un résultat négatif indique une augmentation de la concentration en IL-6 ou IL-8 et un résultat positif indique une diminution de la concentration en IL-6 ou IL-8 en illustrant une protection de la cellule ou une réparation plus rapide des dégâts causés par les UV. Une protection de 100% correspond idéalement à la suppression totale des effets délétères des UV sur les cellules de la peau.

Les résultats pour les interleukines 6 sont présentés sur la figure 1 et pour les interleukines 8 sur la figure 2.

Sur ces figures, la référence 1 correspond au traitement par la niacinamide uniquement à une concentration de 1,2 mg/ml, les références 2 correspondent à un traitement par une illumination à 660 nm seule pour chacun des 10 programmes d'illumination indiqués dans le tableau 1, et les références 3 correspondent à un traitement selon le procédé de l'invention combinant les effets de la niacinamide à une concentration de 1,2 mg/ml et d'une illumination à 660 nm pour chacun des 10 programmes d'illumination indiqués dans le tableau 1.

On constate que quelque soit le programme d'illumination, le procédé de l'invention présente un effet bénéfique sur les cellules exposées au UV par rapport au cellules exposées aux UV et/ou qui n'ont été soumises qu'à la niacinamide. Ces effets bénéfiques sont particulièrement visibles sur les mesures de la concentration des interleukines-8. On constate également que si certains programmes d'illumination associés à un traitement par la seule illumination à 660nm augmentent également la protection des cellules par rapport à l'application seule de la niacinamide, le procédé de l'invention, pourtous les programmes d'illumination, assure une protection bien plus importante sur les cellules de la peau que pour ces traitements de seule illumination à 660nm.

On s'intéresse à présent au vieillissement cutanée appelé « photo-vieillissement » induits par l'exposition aux UV. L'un des paramètres du vieillissement cutané observé en clinique est l'apparition de rides associées en partie à une diminution de la production de collagène par les cellules du derme, les fibroblastes. Cette diminution peut être accompagnée d'une production accrue des métalloprotéases (MMP) responsables de la dégradation des fibres de collagène existant dans le derme. On mesure la production de pro-collagène 1 produit par les fibroblastes dans les surnageants issus du protocole précédemment définis pour lesquels les cellules ont été illuminées soit sans autre traitement, soit en combinaison avec la niacinamide à une concentration de 0,4 mg/ml, et comparés avec un traitement seul à la niacinamide à une concentration de 0,4 mg/ml.

Les résultats sont présentés sur la figure 3 pour les programmes d'illumination 3 et 8. Comme pour les figures 1 et 2, la référence 1 correspond au traitement par la niacinamide uniquement, les références 2 correspondent à un traitement par une illumination à 660 nm seule pour les programmes 3 et 8 d'illumination indiqués dans le Tableau 1, et les références 3 correspondent à un traitement selon le procédé de l'invention combinant les effets de la niacinamide et d'une illumination à 660 nm pour les programmes 3 et 8 d'illumination indiqués dans le Tableau 1.

On constate une production accrue de pro-collagène 1 induit par le traitement par le procédé de l'invention comparé à un traitement à la niacinamide, et un traitement avec la seule illumination à 660nm.

Les résultats présentés précédemment démontrent la synergie inattendue entre la niacinamide et une illumination à une longueur d'onde de 660nm sur l'effet anti-inflammatoire du procédé sur les kératinocytes et l'accroissement de la production de pro-collagène 1, illustrant ainsi les effets de protection et anti-vieillissement de la peau du procédé de traitement cosmétique de l'invention.

Cette combinaison entre l'application de la niacinamide et une illumination par une lumière dite rouge à une longueur d'onde centrée sur 660nm, soit entre 620 et 690 nanomètres, n'est pas démontrée dans l'art antérieur cité plus haut. Cette combinaison est de nature synergique au regard de ses effets précédemment démontrés en référence aux figures 1 à 3 notamment, qui vont au-delà de la somme des effets de la niacinamide d'une part, et d'une illumination à 660nm d'autre part. Cette synergie ne pouvait pas être rendue évidente par la seule connaissance de la niacinamide d'un côté et de la lumière rouge de l'autre côté et ce, même si chacun de ces deux traitements était déjà connu pour son effet bénéfique sur la peau dans le cadre de procédés impliquant d'autres longueurs d'onde et/ou d'autre agents actifs.

Le procédé de traitement cosmétique de l'invention peut comprendre, en outre, l'application d'un autre agent actif, la photolyase, et une illumination supplémentaire à une longueur d'onde de 440nm, considérant une largeur de demi-bande de 30nm. D'autres longueurs d'onde et d'autres agents actifs peuvent être également prévus, comme il sera vu plus loin.

Des tests in-vitro sont réalisés pour un traitement selon le procédé de l'invention consistant en l'application d'une composition comprenant de la niacinamide et de la photolyase, et une illumination à 660 nm, 440 nm, et 780 nm ou 940nm. Toutes ces longueurs d'onde sont considérées avec une largeur de demi-bande de 30nm. On s'intéresse plus particulièrement à l'ajout du couple photolyase/illumination à 440nm qui entraîne un changement de la structure tri-dimensionnelle de la photolyase sans autre modification de sa structure chimique.

Concernant l'illumination appliquée au cours de ces tests, 3 programmes différents sont évalués. Pour chaque programme, 3 longueurs d'onde sont appliquées conjointement selon les indications du Tableau 2 ci-dessous.

[Tableaux 2] : Paramètres associés à chaque programme d'illumination

| λ | Programme 1 30 secondes | | Programme 2 15 secondes | | Programme 3 15 secondes | |
|---|---|---|---|---|---|---|
| | Irradiance ($mW/cm^2$) | Fluence ($mJ/cm^2$) | Irradiance ($mW/cm^2$) | Fluence ($mJ/cm^2$) | Irradiance ($mW/cm^2$) | Fluence ($mJ/cm^2$) |
| 440 | 3,8 | 57 | 3,8 | 29 | 7,4 | 56 |
| 660 | 5,3 | 80 | 5,3 | 40 | 10,6 | 80 |
| 780 | - | - | - | - | 4 | 30 |
| 940 | 1,5 | 23 | 1,5 | 11 | - | - |
| TOTAL | 10,6 | 159 | 10,6 | 80 | 22 | 165 |

Des mesures des dégâts de l'ADN par une exposition aux UV sont réalisées par le biais de la quantification des dimères de thymine. Le protocole consiste à observer et mesurer l'aire des dimères de thymine sur des explants de peau exposés aux UVA et UVB sans traitement, avec de la niacinamide et de la photolyase, avec une illumination telle qu'indiquée selon les trois programmes du tableau 2 ci-dessus, et avec une combinaison d'application de niacinamide et de photolyase et d'illumination telle qu'indiquée dans le tableau 2 ci-dessus.

Les résultats sont illustrés sur les figures 4 et 5.

Sur la figure 4, la référence 0 correspond à un témoin UV qui n'a reçu aucun traitement, les référence 1a,1b,1c correspondent au traitement par la niacinamide seule selon, respectivement, les programmes d'illumination 1, 2 et 3 du Tableau 2, et les références 3a,3b et 3c correspondent à un traitement selon le procédé de l'invention combinant les effets de la niacinamide et d'une illumination selon, respectivement, les programmes d'illumination 1,2 et 3 du Tableau 2.

En référence à la figure 5, la photographie 4 rend compte de la présence et de la répartition des dimères de thymine dont la densité traduit la quantité de dégâts liés à une exposition aux UV, cet explant n'ayant subi aucun traitement. La photographie 5 rend compte de la présence et de la répartition des dimères de thymine d'un explant traité par le procédé de l'invention selon le programme 1.

Les résultats présentés montrent une réduction significative de la densité des dimères de thymine d'un explant de peau exposé aux UV et traité par le procédé de l'invention, comparé à un traitement à la niacinamide et à la photolyase et à la seule exposition à une illumination. Ces résultats illustrent ainsi la capacité du procédé de l'invention à réparer les dégâts cosmétiques provoqués par une exposition aux UVs, et de façon plus générale, à régénérer de la peau pour en améliorer l'apparence.

Des tests cliniques ont été menés sur 33 femmes de 35 à 45 ans présentant des phototypes de I à III. Pendant 28 jours, ces femmes ont reçu 6 jours sur 7 le traitement selon le procédé de l'invention. L'illumination correspond au programme 3 présenté sur le Tableau 2. La composition appliquée comporte, en pourcentage massique, 5% de niacinamide (NIACINAMIDE PC), 0,005% de photolyase (PHOTOSOMES V), 0,0005% d'acetyle hexapeptide-8, 0,012% d'extrait d'algues Laminaria, 0,1% de hyaluronate de sodium, 0,076% d'extrait de fruit Caesalpinia spinosa, 0,0,16% d'extrait d'algues Kappaphycus alvarezii, 0,2% de caféine, 0,2% de vitamine C et 0,2% de vitamine E. Le traitement est réalisé par application simultanée de la composition et de l'illumination selon le programme 3. Le traitement selon l'invention est effectué sur une demi-face du visage sur 6 zones du menton au front. L'autre demi-face est traitée par un placebo pour lequel la composition est une crème hydratante. Le choix du coté traité est déterminé de manière aléatoire. Chaque zone est traitée pendant 15 secondes selon le programme 3 du Tableau 3 ; le traitement du visage est ainsi réalisé en 3 minutes.

On évalue les effets du traitement au bout de 28 jours, en comparaison au premier jour de traitement, sur les rides, la densité du derme, l'effet lissant, et la luminosité du teint.

Les figures 6 et 7 illustrent les résultats obtenus concernant la densité du derme. La figure 6 représente six échographies de mesure de la surface non-échogène de la peau prises respectivement au premier jour de traitement (figures 6a,6d), au 28ème jour de traitement par le procédé de l'invention (figures 6c,6f) et au 28ème jour du traitement placebo (figures 6b,6e) pour deux sujets différents (6a,6b,6c ; 6d,6e,6f).

On constate que la zone du visage traitée par le procédé de l'invention chez les deux sujets représentatifs (figures 6c,6f) présentent une réduction significative de la surface noire correspondant à la surface non-échogène ,la bande hypoéchogène sous-épidermique étant une manifestation caractéristique du vieillissement cutané en zone d'exposition solaire chronique. En outre, le nombre de pixel de haute densité a substantiellement augmenté indiquant la réapparition de fibres dermiques plus denses et échogènes. Pour le traitement placebo, chez les deux sujets, la surface non-échogène et le nombre de pixel de haute densité n'ont pas évolués de façon significative.

La figure 7 rend compte, sous forme d'histogramme, du pourcentage moyen de réduction de la surface non-échogène de la peau pour tous les sujets traités au bout de 28 jours de traitement pour, d'une part, un traitement selon le procédé de l'invention (référence 7), et d'autre part le traitement placebo (référence 8).

Les [Fig. 9] et [Fig. 8] illustrent les résultats obtenus concernant la réduction des rides. La profondeur des rides est mesurée par profilométrie. Les figures 8 et 9 rendent compte sous forme d'histogramme, respectivement, de la profondeur moyenne des rides (figure 8), et de la profondeur maximale des rides (figure 9) en moyenne pour tous les sujets traités sur la demi-face du visage traitée par le procédé de l'invention (7a,7b) et sur la demi-face du visage traitée par le placebo (8a,8b) à 14 jours de traitement (7a,8a) et à 28 jours de traitement (7b,8b).

On constate que le placebo aggrave en moyenne les rides de 4%, alors que le traitement de l'invention réduit la sévérité des rides de 6% en moyenne.

La figure 10 illustre les résultats obtenus par observation clinique sur le pourcentage moyen de variation du lissage de la peau engendré par le procédé de traitement de l'invention, les références étant les mêmes que pour les figures 8 et 9 et un pourcentage élevé traduisant un effet lissant plus conséquent. On constate que le procédé de l'invention (références 7a,7b) permet d'obtenir un effet visible lissant de la peau bien plus important que pour le placebo (références 8a,8b), notamment à 28 jours de traitement (référence 7b).

Enfin, certains sujets de l'étude ont constaté une amélioration de l'homogénéité du teint notamment par réduction de l'intensité de tâches résultant de l'exposition de la peau au soleil.

Le procédé de traitement cosmétique de l'invention permet ainsi d'améliorer l'aspect visible de la peau en contrant son vieillissement, comme les résultats présentés précédemment le démontre, et relève d'un traitement cosmétique anti-âge efficace.

L'invention porte en outre sur un dispositif de mise en oeuvre du procédé de l'invention. Le dispositif de l'invention est décrit en référence aux figures 11 et 12.

Le dispositif 10 comprend un boitier 11 de forme générale cylindrique qui comporte un dispositif électroluminescent 12 et une capsule amovible 13 d'application de la composition sur la peau. Les parois du dispositif présentent une partie embossée qui relève exclusivement d'un caractère esthétique. Le dispositif électroluminescent 12 présente principalement une série de diodes électroluminescentes (LEDs) 9 réparties régulièrement et circonférentiellement autour de l'axe principal XX' du coté d'une première extrémité 10a du dispositif 10 et montées sur une carte électronique dédiée 14. Chaque LED 9 est surmontée d'une lentille 15 formant guide de lumière et assurant la focalisation de la lumière émise par les LEDs 9 dans une direction contrôlée comme il sera explicité plus loin. Dans un mode de réalisation préférentiel, neuf LEDs 9 sont agencées circonférentiellement sur la carte électronique 14. Les LEDs sont reliées électriquement à une batterie 16 située au niveau du manche du dispositif 10 du coté de son extrémité opposée 10b.

En référence à la figure 12, le dispositif comporte, en amont des LEDs 9 du côté de sa première extrémité 10a un logement 17 de réception de la capsule amovible 13. Ce logement 17 présente une forme générale tronconique mais peut présenter toute forme adaptée coïncidant avec la structure de la paroi externe de la capsule 13. Deux crochets 18 diamétralement opposés sont ménagés à proximité de l'ouverture 19 du logement 17 pour assurer la solidarisation amovible de la capsule 13 insérée dans le logement de réception 17 selon la flèche F.

En référence à la figure 12, la capsule 13 présente principalement un capuchon 20 amovible relativement à ladite capsule 13, un distributeur 21 sous forme de boule, un réservoir de composition 23 relié au distributeur 21 par au moins une ouverture 22 assurant la distribution de la composition sur la surface de la boule 21, et une paroi de fond 24 mobile en coulissement relativement à la boule 21 entre une position la plus éloignée de la boule 21 dans laquelle la composition reste dans le réservoir 23, et une position la plus proche de la boule 21 dans laquelle la composition, ou une partie de la composition, contenu dans le réservoir 23 est libérée sur la surface de la boule 21. Les parois latérales 25 de la capsule 13 sont translucides ou transparentes pour assurer la diffusion de la lumière sur la surface et dans la direction visée. Plus particulièrement, les lentilles 15 associées aux LEDs 13 sont configurées pour assurer la distribution de la lumière émise dans les parois 25 de la capsule elles-mêmes configurées pour faire converger la lumière au niveau de la boule de distribution 21 en illuminant ainsi la zone de la peau sur laquelle est simultanément appliquée la composition. En outre, la boule de distribution 21 est maintenue en place par une collerette 28 formant extension des parois latérales 25 de la capsules, et qui est également translucide ou transparente. Ainsi, lors de l'illumination, la lumière est distribuée au niveau de la boule de distribution 21 comme précédemment indiqué, mais également par la collerette 28.

Lorsque la capsule 13 est insérée dans le logement de réception 17, la paroi de fond 24 vient en appui de contact contre un piston 26 dont le déplacement axial en direction de la capsule 13 est assuré par un moteur 27 relié à la batterie 16. Ce déplacement axial non représenté du piston 26 pousse donc la paroi de fond 24 de la capsule 13 vers la boule 21 selon une course définie correspondant à la distribution d'une dose de composition, ce qui entraîne la libération de la composition contenue dans le réservoir 23 sur la surface de cette boule de distribution 21, cette dernière étant destiné à venir rouler sur la surface de la peau à traiter et ainsi à appliquer la composition sur la peau. A l'utilisation suivante, le piston 26 entraîne de nouveau la paroi de fond 24 en déplacement axial vers la boule de distribution 21 sur une nouvelle course pour délivrer une nouvelle dose de composition. A titre d'exemple, la capsule peut être configurée pour délivrer sept doses de composition correspondant à une semaine de traitement.

Un système de commande non représenté permet de commander l'illumination par les LEDs et l'actionnement du piston et la délivrance de la composition sur la peau. Préférentiellement, l'illumination et la délivrance de la composition sont commandées simultanément.

L'homme du métier saura adapter le dispositif de l'invention selon le procédé de l'invention appliqué. Par exemple pour une composition comportant de la niacinamide et une illumination à 660nm, les LEDs 9 ou partie des LEDs 9 sont configurées pour émettre à 660nm et la composition contenue dans le réservoir 23 de la capsule 13 comprend de la niacinamide. Pour un procédé de l'invention pour lequel la composition comprend de la niacinamide et de la photolyase et une illumination à 660nm et à 440 nm, les LEDS 9 ou partie des LEDs 9 sont configurées pour émettre respectivement à 660nm et 440nm et la composition contenue dans le réservoir 23 comprend de la niacinamide et de la photolyase. Enfin, lorsque le procédé de l'invention prévoit la composition telle qu'énoncée précédemment dans le cadre des tests cliniques et une illumination à 660nm, 440 nm et 780nm, la composition contenue dans le réservoir 23 comprend les actifs énoncés et les LEDS 9 ou partie des LEDs 9 sont configurées pour émettre respectivement à 660nm et 440nm. Il en est de même lorsque l'illumination prévoit la longueur d'onde de 940 nm.

A titre d'exemple, la figure 13 illustre un mode de réalisation du dispositif électroluminescent de l'invention 12 et plus particulièrement de l'assemblage de LEDs 9a,9b,9c,9d sur la carte électronique 14. Les LEDs sont dans cet exemple au nombre de 9 et réparties circonférentiellement sur la carte électronique 14 autour du piston 26 qui traverse la carte électronique 14, étant entendu que la référence 9a correspond aux LEDs émettant à 660nm, la référence 9b correspond aux LEDs émettant à 440nm, la référence 9c correspond aux LEDs émettant à 940nm et la référence 9d correspond aux LEDs émettant à 780 nm. Le système de commande non représenté peut activer tout ou partie des LEDs, et ainsi mettre en oeuvre soit un procédé selon l'invention impliquant une illumination à 660 nm seulement, soit un procédé selon l'invention impliquant une illumination selon l'un des trois programmes d'illumination présentés sur le Tableau 2, soit encore une illumination avec les quatre longueurs d'ondes précitées.

## Revendications

1. Procédé de traitement cosmétique non thérapeutique comprenant au moins les étapes de :

   - application sur la peau d'un sujet d'une composition active comprenant au moins de la niacinamide, et

- illumination de la peau par au moins une source lumineuse émettant à une longueur d'onde comprise entre 620 et 690 nanomètres, de préférence de 660 nanomètres,

l'illumination étant réalisée avant, simultanément ou après l'application de la composition.

2. Procédé selon la revendication précédente, **caractérisé en ce que** la composition active comprend en outre de la photolyase.

3. Procédé selon l'une quelconque des revendications 1 et 2, **caractérisé en ce que** l'illumination de la peau est en outre réalisée par au moins une source lumineuse émettant à une longueur d'onde comprise entre 410 et 470 nanomètres, de préférence de 440 nanomètres.

4. Procédé selon la revendication précédente, **caractérisé en ce que** l'illumination de la peau est outre réalisée par au moins une source lumineuse émettant à une longueur d'onde comprise entre 750 et 810 nanomètres, de préférence de 780 nanomètres.

5. Procédé selon l'une quelconque des revendications 3 et 4, **caractérisé en ce que** l'illumination de la peau est outre réalisée par au moins une source lumineuse émettant à une longueur d'onde comprise entre 910 et 970 nanomètres, de préférence de 940 nanomètres.

6. Procédé selon l'une quelconque des revendications précédentes, **caractérisé en ce que** le pourcentage massique de niacinamide dans la composition est compris entre 0,5 et 10%.

7. Dispositif de traitement cosmétique non thérapeutique comprenant au moins des moyens d'application sur la peau d'un sujet d'une composition active comprenant au moins de la niacinamide, et des moyens d'illumination de la peau par au moins une source lumineuse émettant à une longueur d'onde comprise entre 620 et 690 nanomètres, de préférence de 660 nanomètres,
les moyens d'illumination et d'application de la composition active étant configurés pour que l'illumination puisse être réalisée avant, simultanément ou après l'application de la composition.

8. Dispositif de traitement cosmétique selon la revendication précédente, **caractérisé en ce que** la composition active comprend en outre de la photolyase.

9. Dispositif de traitement cosmétique selon l'une quelconque des revendications 7 et 8, **caractérisé en ce que** les moyens d'illumination de la peau comportent en outre au moins une source lumineuse émettant à une longueur d'onde comprise entre 410 et 470 nanomètres, de préférence de 440 nanomètres.

10. Dispositif de traitement cosmétique selon l'une quelconque des revendications 7 à 9, **caractérisé en ce que** les moyens d'illumination de la peau comportent en outre au moins une source lumineuse émettant à une longueur d'onde comprise entre 750 et 810 nanomètres, de préférence de 780 nanomètres.

11. Dispositif de traitement cosmétique selon l'une quelconque des revendications 9 et 10, **caractérisé en ce que** les moyens d'illumination de la peau comportent en outre au moins une source lumineuse émettant à une longueur d'onde comprise entre 910 et 970 nanomètres, de préférence de 940 nanomètres.

12. Procédé de traitement cosmétique non thérapeutique comprenant la mise en oeuvre d'un dispositif selon l'une quelconque des revendications 7 à 11.

**Patentansprüche**

1. Verfahren zur nicht-therapeutischen kosmetischen Behandlung, das zumindest die folgenden Schritte umfasst:

   - Aufbringen einer Wirkzusammensetzung, die zumindest Niacinamid umfasst, auf die Haut eines Individuums, und
   - Bestrahlen der Haut mit mindestens einer Lichtquelle, die bei einer Wellenlänge im Bereich von 620 bis 690 Nanometer, vorzugsweise von 660 Nanometer, emittiert,

wobei das Bestrahlen vor dem Aufbringen der Zusammensetzung, währenddessen oder danach erfolgt.

2. Verfahren nach einem der vorhergehenden Ansprüche, **dadurch gekennzeichnet, dass** die Wirkzusammensetzung darüber hinaus Photolyase umfasst.

3. Verfahren nach einem beliebigen der Ansprüche 1 und 2, **dadurch gekennzeichnet, dass** das Bestrahlen der Haut darüber hinaus mit mindestens einer Lichtquelle erfolgt, die bei einer Wellenlänge im Bereich von 410 bis 470 Nanometer, vorzugsweise von 440 Nanometer, emittiert.

4. Verfahren nach dem vorhergehenden Anspruch, **dadurch gekennzeichnet, dass** das Bestrahlen der Haut darüber hinaus mit mindestens einer Lichtquelle erfolgt, die bei einer Wellenlänge im Bereich von 750 bis 810 Nanometer, vorzugsweise von 780 Nanometer, emittiert.

5. Verfahren nach einem beliebigen der Ansprüche 3 und 4, **dadurch gekennzeichnet, dass** das Bestrahlen der Haut darüber hinaus mit mindestens einer Lichtquelle erfolgt, die bei einer Wellenlänge im Bereich von 910 bis 970 Nanometer, vorzugsweise von 940 Nanometer, emittiert.

6. Verfahren nach einem beliebigen der vorhergehenden Ansprüche, **dadurch gekennzeichnet, dass** der Massenprozentanteil an Niacinamid in der Zusammensetzung im Bereich von 0,5 bis 10% liegt.

7. Vorrichtung zur nicht-therapeutischen kosmetischen Behandlung, das zumindest Mittel zum Aufbringen einer Wirkzusammensetzung, die zumindest Niacinamid umfasst, auf die Haut eines Individuums und Mittel zum Bestrahlen der Haut mit mindestens einer Lichtquelle umfasst, welche bei einer Wellenlänge im Bereich von 620 bis 690 Nanometer, vorzugsweise von 660 Nanometer, emittiert, wobei die Mittel zum Bestrahlen und zum Aufbringen der Wirkzusammensetzung derart ausgelegt sind, dass das Bestrahlen vor dem Aufbringen der Zusammensetzung, währenddessen oder danach erfolgen kann.

8. Vorrichtung zur kosmetischen Behandlung nach dem vorhergehenden Anspruch, **dadurch gekennzeichnet, dass** die Wirkzusammensetzung darüber hinaus Photolyase umfasst.

9. Vorrichtung zur kosmetischen Behandlung nach einem beliebigen der Ansprüche 7 und 8, **dadurch gekennzeichnet, dass** die Mittel zum Bestrahlen der Haut darüber hinaus mindestens eine Lichtquelle umfassen, die bei einer Wellenlänge im Bereich von 410 bis 470 Nanometer, vorzugsweise von 440 Nanometer, emittiert.

10. Vorrichtung zur kosmetischen Behandlung nach einem beliebigen der Ansprüche 7 bis 9, **dadurch gekennzeichnet, dass** die Mittel zum Bestrahlen der Haut darüber hinaus mindestens eine Lichtquelle umfassen, die bei einer Wellenlänge im Bereich von 750 bis 810 Nanometer, vorzugsweise von 780 Nanometer, emittiert.

11. Vorrichtung zur kosmetischen Behandlung nach einem beliebigen der Ansprüche 9 und 10, **dadurch gekennzeichnet, dass** die Mittel zum Bestrahlen der Haut darüber hinaus mindestens eine Lichtquelle umfassen, die bei einer Wellenlänge im Bereich von 910 bis 970 Nanometer, vorzugsweise von 940 Nanometer, emittiert.

12. Verfahren zur nicht-therapeutischen kosmetischen Behandlung, das den Einsatz einer Vorrichtung nach einem beliebigen der Ansprüche 7 bis 11 umfasst.

**Claims**

1. Nontherapeutic cosmetic treatment process comprising at least the steps of:

   - applying to the skin of an individual an active composition comprising at least niacinamide, and
   - illuminating the skin with at least one light source emitting at a wavelength of between 620 and 690 nanometres, preferably 660 nanometres,

   the illumination being performed before, simultaneously with or after the application of the composition.

2. Process according to the preceding claim, **characterized in that** the active composition also comprises photolyase.

3. Process according to either of Claims 1 and 2, **characterized in that** the illumination of the skin is also performed with at least one light source emitting at a wavelength of between 410 and 470 nanometres, preferably 440 nanometres.

4. Process according to the preceding claim, **characterized in that** the illumination of the skin is also performed with at least one light source emitting at a wavelength of between 750 and 810 nanometres, preferably 780 nanometres.

5. Process according to either of Claims 3 and 4, **characterized in that** the illumination of the skin is also performed with at least one light source emitting at a wavelength of between 910 and 970 nanometres, preferably 940 nanometres.

6. Process according to any one of the preceding claims, **characterized in that** the mass percentage of niacinamide in the composition is between 0.5% and 10%.

7. Nontherapeutic cosmetic treatment device comprising at least means for applying to the skin of an individual an active composition comprising at least niacinamide, and means for illuminating the skin with at least one light source emitting at a wavelength of between 620 and 690 nanometres, preferably 660 nanometres, the means for illuminating and applying the active composition being configured so that the illumination can be performed before, simultaneously with or after the application of the composition.

8. Cosmetic treatment device according to the preceding claim, **characterized in that** the active composition also comprises photolyase.

9. Cosmetic treatment device according to either of Claims 7 and 8, **characterized in that** the means for illuminating the skin also include at least one light source emitting at a wavelength of between 410 and 470 nanometres, preferably 440 nanometres.

10. Cosmetic treatment device according to any one of Claims 7 to 9, **characterized in that** the means for illuminating the skin also include at least one light source emitting at a wavelength of between 750 and 810 nanometres, preferably 780 nanometres.

11. Cosmetic treatment device according to either of Claims 9 and 10, **characterized in that** the means for illuminating the skin also include at least one light source emitting at a wavelength of between 910 and 970 nanometres, preferably 940 nanometres.

12. Nontherapeutic cosmetic treatment process comprising the use of a device according to any one of Claims 7 to 11.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2008031833 A
- US 2007112042 A
- US 2009209600 A
- EP 2861203 A
- US 2018200174 A
- WO 2016146778 A
- EP 3586926 A
- US 2014236265 A